Europäisches Patentamt

European Patent Office

Office européen des brevets

(11) Numéro de publication : **0 385 841 B1**

## (12) FASCICULE DE BREVET EUROPEEN

(45) Date de publication du fascicule du brevet :
**22.06.94 Bulletin 94/25**

(51) Int. Cl.$^5$ : **A61K 35/80, C07K 3/12**

(21) Numéro de dépôt : **90400524.6**

(22) Date de dépôt : **26.02.90**

(54) **Extraits de cyanobactéries filamenteuses, leur procédé de préparation et leur activité en tant que facteur de croissance.**

(30) Priorité : **28.02.89 FR 8902992**

(43) Date de publication de la demande :
**05.09.90 Bulletin 90/36**

(45) Mention de la délivrance du brevet :
**22.06.94 Bulletin 94/25**

(84) Etats contractants désignés :
**AT BE CH DE ES GB GR IT LI LU NL SE**

(56) Documents cités :
**FR-M- 2 674
CHEMICAL ABSTRACTS, vol. 99, 1983, page
345, résumé no. 136869s, Columbus, Ohio, US;
A.C. STEWART et al.: "Extraction and partial
purification of an acidic plastocyanin from a
blue-green alga", & PHOTOBIOCHEM. PHO-
TOBIOPHYS. 1983, 6(2), 67-73
ANN.BOT., vol. 37, no. 152, 1979, pages
737-741; A.B. GUPTA et al.: "Extraction, isolation, and bioassay of a gibberellin-like substance from Phormidium foveolarum"**

(73) Titulaire : **PIERRE FABRE COSMETIQUE
125 rue de la Faisanderie
F-75116 Paris (FR)**
Titulaire : **COMPAGNIE THERMALE DE DAX,
SOCIETE ANONYME d'ECONOMIE MIXTE:
Cours de Verdun
F-40100 Dax (FR)**

(72) Inventeur : **Dubois, Jacques
8, Boulevard Gambetta
F-11100 Narbonne (FR)**
Inventeur : **Lefort-Tran M.
15, rue Grès
F-91990 Gif-Sur-Yvette (FR)**
Inventeur : **Hodara, Marie-Laure
13, rue Péraudel
F-81100 Castres (FR)**

(74) Mandataire : **Morelle, Guy Georges Alain et al
CABINET MORELLE & BARDOU, SC
5, Boulevard de la Méditerranée
F-31400 Toulouse (FR)**

## Description

La présente invention est relative à des extraits de cyanobactéries filamenteuses, à leur procédé de préparation et à leurs applications thérapeutiques et/ou dermo-cosmétiques en raison de leurs effets analogues à ceux d'un facteur de croissance.

L'utilisation des algues est bien connue depuis l'antiquité pour leurs propriétés cicatrisantes. Ainsi, Lesage a utilisé avec succès, en médecine vétérinaire, les propriétés de certaines algues. En 1952, Lefevre a mis en évidence les propriétés cytostimulantes de certaines cyanobactéries. En utilisant un filtrat de culture d'algues, obtenu par filtration stérilisante, il a constaté qu'il obtenait aussi bien sur les cellules végétales en cultures que sur les cellules animales, une stimulation de la multiplication cellulaire. Ces travaux, confirmés par des études pharmacologiques et cliniques, n'ont pas permis alors d'apporter de précisions sur les mécanismes d'action (voir "Cyanostimulines et cicatrisation", M. Lefevre, G. Laporte, O. Flandre, Colloq. Inter. CNRS, Paris, n° 145, 217-227, 1963 et "Sur la sécrétion par certaines cyanophycées de substances stimulant la multiplication cellulaire", C.R. Acad. Sci., t.256, pp. 254-260).

L'invention concerne les extraits de cyanobactéries filamenteuses obtenues à partir de cultures in vitro. Les travaux ont porté sur diverses cyanobactéries filamenteuses Oscillatoria phormidium du type LPP groupe A, isolée dans les bassins de la Compagnie Thermale de Dax.

L'invention a encore pour objet un procédé de préparation des extraits actifs à partir de cultures des espèces de cyanobactéries ci-dessus dénommées. Ces cyanobactéries sont soumises, en vue de libérer, d'extraire, d'isoler et de concentrer la fraction active responsable de l'activité, aux traitements suivants.

Schéma du procédé :

**Extrait Brut A**

Traitement 100°C - 10 mn
Centrifugation

Culot      **Extrait Thermostable B (surnageant)**

Précipitation milieu
alcoolique 60 %
Centrifugation

Surnageant C/S      **Précipité C/P**

1. Préparation de l'Extrait Brut A.

Après lavage des filaments et rinçage à l'eau distillée, on procède à un craquage des cellules par sonication. Une première centrifugation est effectuée pendant une heure (10 000 g) puis le surnageant est recueilli et est à nouveau centrifugé pendant une heure (10 000 g).

Le surnageant, selon le degré de purification souhaité, peut alors être soumis à une dialyse afin d'éliminer les constituants de poids moléculaire inférieur à 10 Kd, puis à une lyophilisation.

On obtient la fraction A de couleur bleue due à des pigments de type Phycobilines, et qui présente les caractéristiques suivantes :
- Spectre : maximum d'absorption à 610 nm ;
- Microanalyse : N = 12 % et O = 33 % ;
- Rendement moyen : 0,02 à 0,10 g par litre de milieu de culture.

2. Préparation de l'Extrait B.

L'extrait brut A lyophilisé est remis en solution aqueuse à 1 %. La solution est alors chauffée à 100°C pendant 10 minutes afin de provoquer par la dénaturation thermique la précipitation d'une fraction des constituants de l'extrait A. 70 % des protéines sont ainsi éliminées.

Le liquide surnageant est récupéré par centrifugation (10 000 g) et lyophilisé. On obtient ainsi l'extrait thermostable B avec un rendement de 25 % par rapport à l'extrait A. Cet extrait présente les caractéristiques physico-chimiques suivantes :

- Spectre visible : absence d'absorption à 610 nm ;
- Microanalyse :

N = 4 % (12 % dans l'extrait A)

O = 46 % (33 % dans l'extrait A).

Cette analyse témoigne d'un enrichissement de l'extrait B en fractions osidiques.

L'électrophorèse sur gel d'Acrylamide, gradient 6/15 %, en présence de Sodium dodecyl sulfate, après révélation par le Bleu de Comassie, permet de caractériser des bandes de polypeptides de l'Extrait B.

Par rapport à l'extrait A il y a disparition des bandes spécifiques des pigments bleus et un enrichissement dans la bande spécifique des poids moléculaires élevés.

La nature glycoprotéique de certaines fractions est révélée par leur coloration avec le réactif de Schiff.

3. Préparation de l'Extrait C.

L'extrait B lyophilisé est remis en solution aqueuse à 1 %. 10 ml de cette solution, maintenus à une température de 0°C, sont additionnés de 15 ml d'alcool éthylique à 90°(correspondant à une précipitation en milieu alcoolique à 66°).

Par centrifugation (10 000 g) pendant 30 minutes, le précipité recueilli forme l'extrait C ; il représente 30 par rapport à l'extrait B.

Ses caractéristiques physico-chimiques sont les suivantes :
- Micro-analyse : N = 5 % (léger enrichissement par rapport à l'extrait B) ;
- Electrophorèse : elle permet d'établir la nature glycoprotéique des constituants. En effet, une fraction importante de nature protéique a été éliminée avec le surnageant.

Etude toxicologique

Cette étude a mis en évidence la parfaite tolérance locale et générale des extraits de l'invention et leur absence de toxicité in vitro et in vivo.

Administrés aussi bien en applications locales sur la peau et les muqueuses qu'en injections intradermiques, ils n'ont provoqué aucune réaction ni aucun phénomène tel que rougeur, inflammation ou allergie.

Etude pharmacologique

a. Evaluation de l'effet stimulant de croissance in vitro.

L'activité des différents extraits de l'invention a été mesurée par le test de croissance sur Fibroblastes murin L 929 (Adolphe, M., Pointet, Y., Renot, X., Wepierre, J. "Use of fibroblast cell culture for the study of wound healing drugs", Int. J. Cosmetic Science, 6, 55-58, 1984, modifiée par Hodara et Lefort-Tran, 1986).

Le produit à tester est ajouté dans le milieu de culture carencé en sérum de veau nouveau-né et le pourcentage relatif de croissance est évalué par rapport au témoin (milieu enrichi par 0,5 % de sérum de veau).

1. Extrait A : à la concentration de 200μg/ml, on obtient un pourcentage relatif de croissance de 144 %.

2. Extrait B : à la concentration de 200μg/ml, le pourcentrage obtenu est de 145 %.

3. Extrait C : à la concentration de 100μg/ml, le pourcentage est de 145 %.

Ce test permet de constater que l'extrait B conserve intégralement son activité après traitement par la chaleur et que la précipitation en milieu alcoolique enrichit de manière considérable l'extrait C en principe actif.

Activité sur la prolifération cellulaire

Les essais ont permis de mettre en évidence l'activité biologique des extraits de l'invention.

En premier lieu, les extraits possèdent une activité sur la croissance cellulaire qu'ils stimulent. Cet effet, est comparable à celui obtenu avec le sérum de veau nouveau-né ; il exprime la présence dans les extraits d'une substance assimilable à un facteur de croissance.

L'analyse en cytométrie de flux de la population cellulaire qui permet de répartir et de dénombrer cette population en fonction des différentes phases classiques du cycle (GO, S, G1 et M) a montré une nette accélération du passage de la phase G1 à la phase S, ceci confirme l'effet stimulant de l'extrait sur une phase précise du cycle.

## Activité réparatrice sur la morphologie des cellules fibroblastiques

L'effet stimulant de la croissance s'accompagne d'une activité réparatrice sur les cellules des cultures carencées en sérum, qui subissent des modifications importantes au niveau morphologique (cellules à plusieurs prolongements). L'addition de l'extrait entraîne un retour rapide à la forme en fuseau.

Evalué sur les cultures asynchrones et synchrones, cet effet sur la morphologie se manifeste par une modification de la répartition de la population cellulaire :
- diminution des classes de cellules à 3, 4 ou 5 prolongements ;
- augmentation de la classe des cellules fusoïdes ;
- forte augmentation des mitoses dès 24 heures, caractérisée par le nombre élevé de cellules arrondies.

Cet effet sur la morphologie cellulaire a été confirmé par l'observation du réseau d'actine constituant du cytosquelette et facteur essentiel de l'acquisition et de la conservation des formes des cellules euraryotiques. Il a été constaté, dans les cellules cultivées en présence de 0,5 % de sérum de veau nouveau-né, la désorganisation du réseau d'actine et l'effet réparateur de l'extrait de l'invention.

De nombreux essais ont été effectués sur différents types de cellules autres que les Fibroblastes murin L 929. Les mêmes actions stimulante et réparatrice ont été mises en évidence.

## Activité agrégative

Il a été constaté en outre que l'extrait A de l'invention induit sur les cultures de fibroblastes, une tendance agrégative mise en évidence par une analyse statistique de la répartition cellulaire.

Cette tendance au regroupement cellulaire en présence d'extrait pourrait être due à une modification de la motilité cellulaire et cette propriété pourrait être utilisée pour prévenir la migration des cellules transformées.

Cette activité est le signe de la présence d'un facteur thermolabile de nature protéique ; elle est faible ou nulle avec les extraits B ou C.

### b. Evaluation de l'activité in vivo.

L'activité des extraits de l'invention a été mise en évidence chez le rat et le lapin.

On a ainsi constaté leur action stimulante sur les tissus nécrosés présentant un retard de cicatrisation. On observe une accélération de la réduction de la surface de la plaie et une reconstitution rapide des tissus cutanés nomraux en même temps qu'une atténuation des phénomènes inflammatoires.

Les études qui viennent d'être rapportées ont mis en évidence la bonne tolérance des extraits de l'invention ainsi que leurs intéressantes propriétés qui les rendent très utiles en thérapeutique humaine et vétérinaire ainsi qu en dermo-cosmétique.

Selon le but recherché, on pourra utiliser les extraits A et B, doués de la même activité, ou l'extrait C, deux fois plus actif.

Les compositions de l'invention peuvent être présentées sous forme de pommades, crèmes ou lotions pour l'administration locale, en association avec des excipients thérapeutiquement administrables. Dans ces formes, le pourcentage en principe actif est compris entre 0,01 % et 10 %, de préférence 0,2 à 1 %.

## Exemples de formulation :

### Gel topique

| | |
|---|---|
| Extrait A | 0,1 g |
| Methycellulose | 3 g |
| Eau purifiée qsp | 100 g |
| Conservateurs, parfum | QS |

### Solutions injectable-intradermique :

| | |
|---|---|
| Extrait C | 0,5 mg |
| Soluté physiologique | 1 ml |
| Préparation stérile (filtration stérilisante) | |

EP 0 385 841 B1

Crème grasse

Extrait B                          0,5 g
Eau purifiée                       5 ml
Lano-vaseline qsp                  100 g
Conservateurs, parfum     QS
(appliquée tel quel ou tulle gras)

Emulsion

Extrait B                          0,2 g
Monostearate de PEG 1500           5 g
Monostearate de PEG 300            2 g
Huile de paraffine fluide          5 g
Eau purifiée qsp                   100 g
Conservateurs, parfum     QS

Les compositions de l'invention sont utilisées avec profit. Elles sont plus particulièrement indiquées :
a. en dermo-cosmétique, dans les applications suivantes :
- régénération cellulaire : vieillissement cutané, rides ;
- soins corporels : vergétures, raffermissement.
b. en thérapeutique, aussi bien chez l'adulte que chez l'enfant, dans les applications suivantes :
- réparation tissulaire : érythème solaire, piqûres ;
- inflammations superficielles : UV, radiothérapie, acné ;
- cicatrisation des plaies : ulcères, escarres, plaies torpides, retard de cicatrisation, brûlures ;
- en ophtalmologie : plaies et brûlures de la cornée, ulcères tromatiques, kératites ;
- stimulation des défenses : infections et surinfections microbiennes et virales.
c. en laboratoire, comme facteur de croissance pour les cultures cellulaires ou tissulaires.

**Revendications**

**Revendications pour les Etats contractants suivants : AT, BE, CH, DE, GB, IT, LI, LU, NL, SE**

1. Extrait de cyanobactéries filamenteuses Oscillatoria phormidium du type LPP groupe A,
   CARACTERISE EN CE QUE ledit extrait est obtenu à partir :
   - d'un premier extrait préparé par craquage des cellules desdites cyanobactéries à l'aide des ultrasons et centrifugation,
   - ledit premier extrait, mis en solution aqueuse, étant chauffé à 100 °C pendant 10 minutes et soumis à centrifugation pour produire un deuxième extrait,
   - ledit deuxième extrait, mis en solution aqueuse, étant traité par précipitation alcoolique et centrifugation pour obtenir un dernier extrait.

2. Composition CARACTERISEE EN CE QU'elle contient, à titre de principe actif, un extrait selon la Revendication 1.

3. Composition selon la Revendication 2, CARACTERISEE EN CE QU'elle est présentée sous une forme appropriée à l'administration locale ou générale.

4. Composition selon la Revendication 2, CARACTERISEE EN CE QUE la concentration en principe actif est comprise entre 0,01 % et 10 %.

**Revendications pour les Etats contractants suivants : ES, GR**

1. Procédé de préparation d'un extrait de cyanobactéries filamenteuses Oscillatoria phormidium du type LPP groupe A, CARACTERISE EN CE QU'il consiste :
   - à préparer un premier extrait par craquage des cellules desdites cyanobactéries à l'aide des ultrasons et centrifugation,
   - à mettre ledit premier extrait en solution aqueuse, à chauffer ledit premier extrait à 100°C pendant

5

10 minutes et à le soumettre à centrifugation pour produire un deuxième extrait,
- à mettre ledit deuxième extrait en solution aqueuse, à le traiter par précipitation alcoolique et centrifugation pour obtenir un dernier extrait.

**Patentansprüche**

**Patentansprüche für folgende Vertragsstaaten : AT, BE, CH, DE, GB, IT, LI, LU, NL, SE**

1.  Extrakt von faserigen Cyanobakterien Oscillatoria phormidium der Art LPP, Gruppe A, dadurch gekennzeichnet, daß dieser Extrakt erhalten wird ausgehend von:
    - einem ersten Extrakt, der durch Zerbrechen der Zellen der Cyanobakterien mit Hilfe von Ultraschall und Zentrifugation hergestellt wird,
    - wonach der erste, in eine wäßrige Lösung gegebene Extrakt über 10 Minuten auf 100°C erhitzt wird und einer Zentrifugation unterworfen wird, um einen zweiten Extrakt zu ergeben,
    - wonach der zweite, in eine wäßrige Lösung gegebene Extrakt mit einer alkoholischen Fällung und Zentrifugation behandelt wird, um einen letzten Extrakt zu erhalten.

2.  Zusammensetzung, dadurch gekennzeichnet, daß sie als aktiven Inhaltsstoff einen Extrakt nach Anspruch 1 enthält.

3.  Zusammensetzung nach Anspruch 2, dadurch gekennzeichnet, daß sie in einer Form vorliegt, die für die lokale oder generelle Verabreichung geeignet ist.

4.  Zusammensetzung nach Anspruch 2, dadurch gekennzeichnet, daß die Konzentration an aktivem Inhaltsstoff im Bereich zwischen 0,01 % und 10 % liegt.

**Patentansprüche für folgende Vertragsstaaten : ES, GR**

1.  Verfahren zur Herstellung eines Extraktes von faserigen Cyanobakterien Oscillatoria phormidium der Art LPP, Gruppe A, dadurch gekennzeichnet, daß es beruht:
    - auf dem Herstellen eines ersten Extraktes durch Zerbrechen der Zellen der Cyanobakterien mit Hilfe von Ultraschall und Zentrifugation,
    - auf dem Zugeben des ersten Extraktes zu einer wäßrigen Lösung, dem Erhitzen des ersten Extraktes über 10 Minuten auf 100°C und dem Unterwerfen dieses einer Zentrifugation, um einen zweiten Extrakt zu ergeben,
    - auf dem Zugeben des zweiten Extraktes zu einer wäßrigen Lösung, auf dem Behandeln dieses mit einer alkoholischen Fällung und Zentrifugation, um einen letzten Extrakt zu erhalten.

**Claims**

**Claims for the following Contracting States : AT, BE, CH, DE, GB, IT, LI, LU, NL, SE**

1.  Filamentous cyanobacteria extract Oscillatoria phormidium of the group A LPP type, characterised in that said extract is obtained from:
    - a first extract prepared by cracking cells of said cyanobacteria by means of ultrasound and centrifuging,
    - said first extract, placed in an aqueous solution, being heated to 100°C for 10 minutes and subjected to centrifuging in order to produce a second extract,
    - said second extract, placed in an aqueous solution, being treated by alcoholic precipitation and centrifuging in order to obtain a last extract.

2.  Composition characterised in that it contains, as active principle, an extract according to Claim 1.

3.  Composition according to Claim 2, characterised in that it is presented in a suitable form for local or general administration.

4.  Composition according to Claim 2, characterised in that the concentration of active principle is comprised between 0.01% and 10%.

**Claims for the following Contracting States : ES, GR**

1. Method for the preparation of an extract of filamentous cyanobacteria Oscillatoria phormidium of the group A LPP type, characterised in that it consists:
   - of preparing a first extract by cracking cells of said cyanobacteria by means of ultrasound and centrifugation,
   - of placing said first extract in an aqueous solution, of heating said first extract at 100°C for 10 minutes and of subjecting it to centrifugation in order to produce a second extract,
   - of placing said second extract in an aqueous solution, of treating it by alcoholic precipitation and centrifugation in order to obtain a last extract.